Europäisches Patentamt

(19) European Patent Office

Office européen des brevets

(11) EP 0 561 088 B1

## (12) FASCICULE DE BREVET EUROPEEN

(45) Date de publication et mention
de la délivrance du brevet:
**05.02.1997 Bulletin 1997/06**

(51) Int. Cl.⁶: **C08B 30/18**, C12P 19/22,
A23L 1/09, A23L 1/236

(21) Numéro de dépôt: 92403559.5

(22) Date de dépôt: 28.12.1992

(54) **Hydrolysat d'amidon hydrogéné hypocariogène, procédé de préparation et application de cet hydrolysat**

Hydriertes Stärkehydrolysat, das die Kariesbildung vermindert, Verfahren zu seiner Herstellung und seine Anwendung

Low-cariogenic hydrogenated starch hydrolysate, process for obtaining the same and its use

(84) Etats contractants désignés:
AT BE CH DE DK ES FR GB GR IE IT LI LU MC NL
PT SE

(30) Priorité: **19.03.1992 FR 9203312**

(43) Date de publication de la demande:
**22.09.1993 Bulletin 1993/38**

(73) Titulaire: **Roquette Frères**
**F-62136 Lestrem (FR)**

(72) Inventeur: **Caboche, Jean-Jacques**
**F-62400 Bethune (FR)**

(74) Mandataire: **Boulinguiez, Didier et al**
**Cabinet Plasseraud**
**84, rue d'Amsterdam**
**75009 Paris (FR)**

(56) Documents cités:
**EP-A- 0 094 292**          **EP-A- 0 171 964**
**EP-A- 0 368 451**          **WO-A-85/02758**
**US-A- 4 346 116**

• **CHEMICAL ABSTRACTS, vol. 38, no. 7, 1944,**
**Columbus, Ohio, US; abstract no. 1658-89,**
**column 1658 ;**

**Description**

L'invention a pour objet un hydrolysat d'amidon hydrogéné hypocariogène, utilisable comme agent édulcorant ou comme agent de texture dans les produits destinés à être ingérés par les hommes ou les animaux, c'est-à-dire notamment dans les produits alimentaires et dans certains produits pharmaceutiques ou vétérinaires.

L'invention vise également le procédé de préparation dudit hydrolysat, ainsi que l'application de cet hydrolysat dans les produits destinés à être ingérés par les hommes ou les animaux.

Par l'expression "produits destinés à être ingérés par les hommes ou les animaux", on entend les produits destinés à l'ingestion et à l'administration orale tels que diverses denrées alimentaires comme les confiseries, les pâtisseries, les crèmes, les boissons, les confitures, les sauces, les crèmes glacées, les aliments préparés pour animaux, ainsi que les produits pharmaceutiques, vétérinaires, diététiques ou hygiéniques, tels que par exemple les élixirs et les sirops contre la toux, les tablettes ou les comprimés, les pâtes à mâcher, les chewing-gums, les pastilles, les solutions d'hygiène buccale, les pâtes dentifrice.

Par l'expression "hydrolysat d'amidon hydrogéné hypocariogène" on entend un hydrolysat d'amidon hydrogéné qui présente une moindre acidification par les bactéries de la bouche que les sucres classiques tels que le saccharose, le glucose ou le fructose.

De tels hydrolysats d'amidon hydrogénés hypocariogènes sont déjà connus. On peut ainsi citer par exemple les sirops de maltitol à environ 50-55 % et à environ 72-78 % de maltitol sur matière sèche. De tels sirops sont ainsi commercialisés par exemple respectivement sous les marques "LYCASIN 80/55" et "MALTISORB 75/75" par la Société Demanderesse. On peut également mentionner les sirops de maltitol vendus sous les marques "MALTIDEX 100", "MALTIDEX 200", "MALBIT", "FINMALT".

Cependant ces hydrolysats d'amidon hydrogénés hypocariogènes ne sont pas satisfaisants sur tous les plans.

Les problèmes rencontrés peuvent être de plusieurs types.

Ainsi, par exemple, on peut reprocher un manque de pouvoir sucrant à certains de ces sirops ou aux articles finis dans lesquels ils sont utilisés.

Parfois également, la viscosité de ces sirops est insuffisante et les articles finis dans la constitution desquels ils entrent manquent alors de "corps" ou de texture. On est alors tenu d'ajouter des agents viscosifiants tels que la carboxylméthyl cellulose (CMC), ou des gommes végétales dont l'incorporation est difficile.

D'autres inconvénients, de nature essentiellement technologique, s attachent à l'utilisation de certains hydrolysats d'amidon hydrogénés hypocariogènes lorsqu'ils sont utilisés par exemple pour la fabrication de bonbons au sucre cuit. Le premier de ces inconvénients réside en un phénomène d'écoulement ou de fluage (ou phénomène dit de "cold flow") dont la matière constitutive du bonbon est le siège. Ce phénomène, qui se révèle assez lent à 20°C mais qui s'accélère avec l'élévation de température, se traduit à la longue par une déformation des bonbons, préjudiciable à leur commercialisation. Le second inconvénient réside dans le caractère hygroscopique non négligeable de ces bonbons, dû au fait que les produits de confiserie doivent être amenés à une teneur en eau inférieure à 2 %, de préférence inférieure à environ 1 %, ce qui entraîne une tendance à la reprise d'eau au stockage.

On peut également reprocher à certains de ces hydrolysats d'amidon hydrogénés de ne pas être suffisamment hypocariogènes. Il est bien évident que l'on exige des propriétés hypocariogènes beaucoup plus marquées dans une application telle que la fabrication de bonbons au sucre cuit qui, de par leur essence même, risquent d'être en long contact avec les dents, que dans une application telle que par exemple la fabrication de produits liquides très dilués.

Des hydrolysats d'amidon hydrogénés très spécifiques ont ainsi été élaborés et développés par la Société Demanderesse, tout particulièrement pour cette application dans les bonbons au sucre cuit, ces hydrolysats ayant la particularité de pouvoir être utilisés directement pour la fabrication desdits bonbons tout en présentant des propriétés hypocariogènes tout à fait satisfaisantes, leur permettant même de pouvoir être qualifiés de "non cariogènes" dans des pays comme la Suisse où des tests officiels de détermination de la non-cariogénicité des produits ont été mis en place. Les propriétés hypocariogènes optimales de ces hydrolysats, commercialisés sous la marque "LYCASIN 80/55", peuvent être obtenues grâce au respect d'une teneur en polysaccharides hydrogénés de DP supérieur à 20 inférieure à 3 %, et de préférence inférieure à 1,5 %, à côté d'une teneur choisie en produits hydrogénés de DP1 et DP2. Ces hydrolysats d'amidon hydrogénés spécifiques sont décrits par exemple dans les brevets US n° 4 279 931 et 4 346 116.

Des propriétés hypocariogènes très satisfaisantes peuvent également être obtenues sans aucun problème avec des hydrolysats présentant une haute teneur en maltitol, comme c'est le cas par exemple des hydrolysats commercialisés par la Société Demanderesse sous la marque "MALTISORB 75/75". Mais ces hydrolysats ne peuvent être utilisés de façon satisfaisante dans certaines applications comme celle justement des bonbons au sucre cuit, des gelées, des sirops contre la toux, où l'on rencontre un autre inconvénient, qui réside dans les risques de cristallisation encourus lors de l'utilisation de ces hydrolysats.

Tous ces inconvénients - manque de pouvoir sucrant, mauvaises aptitudes technologiques, propriétés hypocariogènes insuffisantes, risques de cristallisation - ne se rencontrent pas simultanément dans un même hydrolysat d'amidon hydrogéné. Mais le problème réside dans le fait que l'atténuation de certains inconvénients entraîne l'aggravation des autres.

Ainsi, par exemple, on peut obtenir des hydrolysats d'amidon hydrogénés (que l'on dénommera dans la suite du texte H.A.H) qui présentent un bon pouvoir sucrant et des propriétés hypocariogènes satisfaisantes. C'est le cas des H.A.H à teneur élevée en maltitol. Mais ces hydrolysats présentent alors des risques de cristallisation ou manquent de viscosité et ne sont pas utilisables dans certaines applications majeures comme la fabrication des bonbons.

Inversement, on peut obtenir des H.A.H qui présentent une bonne viscosité - conférant ainsi aux articles dans lesquels ils sont utilisés un "corps" ou une texture convenables - et qui sont susceptibles d'être utilisés par exemple pour la fabrication de bonbons sans occasionner de problèmes de fluage ou d'hygroscopicité, mais malheureusement ces hydrolysats manquent alors cruellement de pouvoir sucrant et surtout ne présentent pas les propriétés hypocariogènes requises.

Comme cela a été mentionné précédemment, la Société Demanderesse s'est déjà livrée à d'importantes recherches afin d'essayer de résoudre ce problème technique et a développé en particulier les hydrolysats d'amidon hydrogénés connus sous la marque "LYCASIN 80/55". Mais, bien que ces hydrolysats aient représenté un progrès décisif, qui s'est d'ailleurs traduit par un succès commercial important, ils ne demeurent toujours que le résultat d'un compromis entre les propriétés contradictoires dont il a été question ci-dessus et présentent de ce fait encore partiellement les inconvénients que sont le manque de pouvoir sucrant et des aptitudes technologiques relativement limitées dans certaines applications.

Consciente de ces problèmes, la Société Demanderesse s'est efforcée de les atténuer en proposant, dans le brevet européen n° 0 094 292, d'incorporer aux hydrolysats d'amidon hydrogénés destinés à la fabrication des bonbons au sucre cuit une quantité efficace de gomme arabique ou de carboxyméthylcellulose, ainsi qu'éventuellement une quantité de mannitol pouvant aller jusqu'à 10 % en poids.

Cependant, s'il est vrai que cette adjonction aux H.A.H de gomme arabique et/ou de carboxyméthylcellulose, et éventuellement de mannitol, a permis d'améliorer leurs performances dans la fabrication des bonbons au sucre cuit, sur le plan de la reprise en eau et du fluage, il n'en demeure pas moins que des progrès doivent encore être faits. En particulier, en ce qui concerne l'optimisation du procédé de fabrication des bonbons, il serait intéressant de diminuer encore les durées de cuisson et les durées de fabrication, de manière à pouvoir utiliser le matériel traditionnel de confiserie et non plus des installations mises spécialement au point pour la confiserie sans sucre. Il est clair que cela se traduirait en effet par une diminution des coûts de revient. De plus, lors de la cuisson des H.A.H, en vue de la fabrication des bonbons, l'emploi de produits comme la gomme arabique ou la carboxyméthylcellulose, ayant une viscosité élevée en solution, rend parfois difficile l'application du vide en raison d'une tendance manifeste au moussage. D'autre part, l'addition de gomme arabique et/ou de CMC et/ou de mannitol oblige l'utilisateur à procéder à des mélanges supplémentaires, rendant cette technique peu pratique et plus onéreuse.

Enfin, de tels produits peuvent avoir des incidences néfastes sur les articles de confiserie eux-mêmes, à savoir: le développement d'une coloration brune et d'un goût de caramel indésirables. Ces inconvénients ne se rencontrent pas seulement dans les bonbons au sucre cuit mais également, quoique à un degré moindre, dans des articles comme les gommes, les gelées ou les pâtes à mâcher.

On recherche donc toujours à l'heure actuelle un hydrolysat d'amidon hydrogéné qui présente un pouvoir sucrant élevé, ne nécessitant donc pas obligatoirement l'addition d'édulcorants de synthèse, qui ne présente pas de risques de cristallisation, qui présente d'excellentes propriétés hypocariogènes, qui présente de bonnes aptitudes technologiques dans tous types d'application - et en particulier dans la fabrication d'articles nécessitant une cuisson - qui permet de conférer aux articles dans lesquels il est utilisé un "corps" et une texture suffisants, qui ne nécessite pas l'adjonction d'additifs ou de produits auxiliaires, et enfin qui puisse être obtenu à partir d'amidon.

La Société Demanderesse a eu le mérite de concilier tous ces objectifs réputés jusqu'alors inconciliables en élaborant, au prix de nombreuses recherches, une composition tout à fait spécifique d'hydrolysat d'amidon hydrogéné.

Les hydrolysats d'amidon hydrogénés conformes a l'invention sont ainsi caractérisés par le fait qu'ils présentent, les teneurs étant exprimées en poids par rapport à la matière sèche de l'hydrolysat :

- une teneur de 0,1 % à 19 % en sorbitol
- une teneur de 35 % à 87 % de maltitol
- une teneur de 1 % à 17 %, de préférence de 1,5 % à 15 %, et plus préférentiellement encore de 2 à 12 % en poids de polysaccharides non hydrolysables par l'amyloglucosidase dans un test F décrit ci-après,

le complément à 100 % étant constitué par des oligo- ou des poly-saccharides hydrogénés.

Grâce à la présence de la teneur choisie en sorbitol et surtout en maltitol, les hydrolysats d'amidon hydrogénés conformes à l'invention présentent un pouvoir sucrant acceptable, qui peut même être élevé dans le cas où la teneur en maltitol dépasse environ 65 %; et grâce à la présence de la teneur sélectionnée en polysaccharides non hydrolysables par l'amyloglucosidase, ils présentent des aptitudes technologiques tout à fait satisfaisantes, permettent de conférer aux articles dans lesquels ils sont incorporés un "corps" et une texture convenables, ne cristallisent pas, et présentent des propriétés hypocariogènes excellentes.

Afin de déterminer sur les hydrolysats la teneur en produits non hydrolysables par l'enzyme amyloglucosidase, on utilise le test F qui correspond en fait au test de détermination des "fibres alimentaires totales" développé par la Société SIGMA Chemical Company (P.O. BOX 14508, St Louis, MO 63178 USA) et qui est décrit dans le bulletin technique SIGMA No TDFAB-1, de juin 1991. Ce test consiste essentiellement à déterminer la quantité de matière contenue dans l'hydrolysat, non hydrolysable par une amyloglucosidase en présence d'une $\alpha$-amylase thermorésistante et d'une protéase. Cette quantité est exprimée en pourcentage par rapport à une quantité de 1 g environ d'hydrolysat préalablement séché sous vide à 70°C pendant une nuit.

Pour conduire ce test, on procède comme suit :

1) On pèse à 0,1 mg près quatre échantillons de 1 g environ d'hydrolysat préalablement séché sous vide et refroidi dans un dessicateur pendant 1 nuit, que l'on introduit dans un bécher forme haute de 400 ml.

2) On ajoute dans chacun des quatre béchers 50 ml d'un tampon phosphate (0,05 M) à pH : 6,0.

3) On ajoute 0,05 ml d'une solution d'alpha-amylase (produit Sigma n° A 3306) dans chacun des béchers, et l'on mélange intimement.

4) On couvre chaque bécher avec un film aluminium avant de les placer dans un bain d'eau bouillante pour les incuber pendant 30 mn à partir du moment où la température atteint dans les béchers 95°C. On agite doucement à intervalles réguliers de 5 minutes.

5) On refroidit les solutions à température ambiante.

6) On ajuste le pH des solutions à 7,5 ± 0,1 par ajout dans chaque bécher de 10 ml de NaOH 0,171N. On vérifie le pH et on l'ajuste éventuellement avec de la soude (0,171 N) ou de l'acide phosphorique (0,205 M).

7) On ajoute 5 mg de protéase en poudre (produit Sigma n° P-3910) à chacun des béchers.

8) On couvre les béchers avec un film d'aluminium et on les incube à 60°C pendant 30 mn sous agitation continue. Le temps d'incubation de 30 mn débute à partir du moment où la température interne des béchers atteint 60°C.

9) On refroidit à température ambiante.

10) On ajoute 10 ml d'H3PO4 0,205 M à chacun des béchers pour ajuster le pH à 4,5 ± 0,2. On vérifie le pH. On ajuste éventuellement avec précaution avec les solutions de soude ou d'acide phosphorique.

11) On ajoute 0,3 ml d'amyloglucosidase (produit Sigma N A. 9913) à chaque bécher.

12) On couvre chacun des béchers avec un film d'aluminium et on incube pendant 30 minutes à 60°C sous agitation continue. Le temps d'incubation de 30 mn débute à partir du moment où la température interne des béchers atteint 60°C.

13) On ajoute 280 ml d'éthanol à 95 % (v/v), préchauffé à 60°C, à chacun des béchers. (éthanol à 95 % v/v : 50 ml d'eau déminéralisée, éthanol absolu q.s.p. 1000 ml à 20°C).

14) On laisse se former un précipité par abandon à température ambiante pendant au moins 60 minutes, ou une nuit (même temps pour chacun des 4 essais).

15) On filtre sous vide, sur creuset de verre fritté et lit de Celite le contenu de chacun des béchers qu'on lave successivement et avec précaution avec :

- trois fois 20 ml d'éthanol à 78 % (v/v) (éthanol à 78 % v/v : 220 ml d'eau déminéralisée, éthanol absolu q.s.p. 1000 ml à 20°C)
- deux fois 10 ml d'éthanol à 95 % (v/v)
- et deux fois 10 ml d'acétone.

16) On sèche les quatre filtres à 70°C sous vide pendant 1 nuit.

17) On refroidit ces filtres dans un dessicateur avant de les peser à 0,1 mg près, considérant ce poids comme la somme du poids de résidu de filtration (polysaccharides non hydrolysables à l'amyloglucosidase, plus protéines plus cendres) et du poids du creuset avec Celite.

18) On détermine les teneurs en protéines de deux des quatre résidus de filtration provenant des quatre essais en procédant selon la méthode Kjeldahl, en utilisant le coefficient de correction 6,25.

19) On détermine les quantités de cendres sur les deux autres résidus de filtration en plaçant les creusets dans un four à 525°C pendant 5 heures.

20) On calcule, comme indiqué dans le Bulletin technique SIGMA, les quantités de polysaccharides non hydrolysables à l'amyloglucosidase pour les quatre essais et on réalise une moyenne de ces quantités que l'on ramène à la moyenne des quantités de matière d'hydrolysat séché à 70°C sous vide pendant une nuit, en tenant compte dans le calcul de résultats de quatre essais à blanc (sans hydrolysat sec), menés en parallèle.

Ce test F constitue une variante du test de détermination des fibres alimentaires totales dans les aliments décrits dans "J. Assoc. Off. Anal. Chem." Vol 68, N° 2, 1985, p 399.

Il présente l'avantage d'être standardisé, de pouvoir être effectué à l'aide d'un kit complet d'analyse et d'être répétable et reproductible.

Ceci étant, on peut également caractériser les hydrolysats d'amidon hydrogénés conformes à l'invention par leur teneur en polysaccharides précipitables dans l'éthanol et non hydrolysables par l'amyloglucosidase, cette teneur étant déterminée d'après un autre test, dénommé A, réalisé sur la fraction des polysaccharides préalablement précipités dans l'éthanol.

Afin de déterminer sur les hydrolysats la teneur en produits précipitables dans l'éthanol et non hydrolysables par l'enzyme amyloglucosidase, on s'y prend comme suit.

Un échantillon de 10 grammes d'hydrolysat d'amidon hydrogéné amené à un Brix de 75 ± 0,2, soit un indice de réfraction d'environ 1,478, est utilisé pour la détermination du taux de polysaccharides précipitables dans l'éthanol. On rappelle que le Brix est une unité de mesure couramment employée dans l'industrie amidonnière, et que le Brix d'un sirop est déterminé très aisément par lecture au réfractomètre. Un Brix de 75 correspond pour les hydrolysats d'amidon hydrogénés visés par l'invention à une matière sèche d'environ 75 %.

L'échantillon de 10 g d'H.A.H à 75 Brix est additionné de 30 cm$^3$ d'eau distillée et de 60 cm$^3$ d'éthanol absolu. Le mélange est laissé reposer pendant 1 heure à 0°C. Il est ensuite centrifugé à 0°C pendant 15 minutes à 10 000 g.

Le culot obtenu est séché en étuve sous vide maintenue à 80°C.

Le poids de précipité obtenu, $p_1$, représente le poids de polysaccharides précipitables dans l'éthanol contenu dans les 10 g d'échantillon de départ.

Afin de déterminer la teneur de l'hydrolysat en polysaccharides précipitables dans l'éthanol et non hydrolysables par l'amyloglucosidase, on utilise un test A, qui consiste à faire subir aux polysaccharides précipités dans l'éthanol obtenus précédemment une attaque enzymatique à l'aide d'une $\alpha$-amylase thermorésistante, d'une protéase et d'une amyloglucosidase, puis à opérer une précipitation des polysaccharides non hydrolysables avec de l'éthanol à 95 %, à filtrer le précipité ainsi obtenu, à laver ce dernier plusieurs fois à l'alcool et à l'acétone, et enfin à déterminer le poids, $p_2$, de résidu obtenu.

Ce test est également décrit dans "J. Assoc. Off. Anal. Chem." vol. 68, n° 2, 1985, p. 399 auquel on pourra se référer.

Les hydrolysats d'amidon hydrogénés conformes à l'invention peuvent ainsi être caractérisés par le fait qu'ils présentent, les teneurs étant exprimées en poids par rapport à la matière sèche de l'hydrolysat :

- une teneur de 0,1 % à 19 % en sorbitol
- une teneur de 35 % à 87 % de maltitol
- une teneur de 1 % à 20 %, de préférence de 1 à 10 %, et plus préférentiellement encore de 1,5 à 8 % en poids de polysaccharides précipitables dans l'éthanol et non hydrolysables par l'amyloglucosidase dans le test A décrit ci-avant,

le complément à 100 % étant constitué par des oligo- ou des polysaccharides hydrogénés.

Selon une réalisation particulière de l'invention, les hydrolysats d'amidon hydrogénés selon l'invention présentent une teneur en polysaccharides précipitables dans l'éthanol et non hydrolysables par l'amyloglucosidase dans le test A comprise entre 2 et 5 % en poids.

De préférence, les hydrolysats conformes à l'invention présentent une teneur en sorbitol comprise entre 1 et 17 % en poids et plus préférentiellement encore comprise entre 2 et 16 % en poids.

Ils présentent de préférence une teneur en maltitol comprise entre 40 % et 82 % en poids et plus préférentiellement encore comprise entre 45 % et 77 % en poids.

Selon une réalisation avantageuse, les hydrolysats d'amidon hydrogènés conformes à l'invention présentent une teneur en produits de degré de polymérisation (DP) égal à 7 comprise entre 0,2 % et 5 %, de préférence entre 0,2 % et 4 % et plus préférentiellement encore entre 0,2 % et 3,5 % en poids.

Selon une réalisation encore plus avantageuse, l'hypocariogénicité des hydrolysats d'amidon hydrogénés conformes à l'invention est encore plus marquée si ceux-ci présentent une teneur en produits de degré de polymérisation (DP) supérieur à 20 hydrolysables par l'amyloglucosidase dans les conditions du test F décrit ci-avant, inférieure à 5 %, de préférence inférieure à 4 %, plus préférentiellement encore inférieure à 3 %.

La composition des H.A.H conformes à l'invention, et en particulier les teneurs en produits de DP1, DP2, DP7 et DP20 peuvent être déterminées de façon tout à fait conventionnelle par chromatographie liquide haute pression.

Pour préparer les hydrolysats d'amidon hydrogénés conformes à l'invention, on s'y prend comme suit ou d'une manière équivalente.

Un préhydrolysat d'amidon d'un DE (Dextrose Equivalent) compris entre 6 et 20, de préférence entre 7 et 12, obtenu par voie acide et/ou enzymatique, est mélangé à au moins une dextrine et/ou un polyglucose, et ce mélange est soumis à un traitement enzymatique comportant au moins l'action d'une $\beta$-amylase, les conditions de ce traitement étant choisies de façon telle que le DE de l'hydrolysat obtenu soit compris entre 35 et 75, de préférence entre 40 et 70, à l'issue de ce traitement, l'hydrolysat obtenu étant ensuite hydrogéné puis purifié de manière connue en soi.

Par le terme "polyglucose", on entend les produits composés majoritairement de liaisons 1-6, obtenus par condensation ou réarrangement, à partir du glucose ou d'un ou plusieurs sucres éventuellement réduits, sous l'action combinée de la chaleur et d'acides dans un milieu presque dépourvu d'eau. De tels polymères ont été décrits de nombreuses fois et peuvent être obtenus par des procédés tels que ceux décrits notamment dans les brevets US 2 436 967, US 3 766 165, US 4 965 354, US 5 051 500, JP 01-12761 et JP 02-163101. De façon avantageuse, ces polymères sont obtenus à partir de glucose et d'acide citrique, éventuellement en présence de sorbitol.

Par le terme "dextrine", on entend les produits obtenus par chauffage de l'amidon amené à un faible taux d'humidité, en présence généralement de catalyseurs acides ou basiques. Ce "grillage à sec" de l'amidon, le plus couramment en présence d'acide, entraîne à la fois une dépolymérisation de l'amidon et une condensation des fragments d'amidon obtenus, et conduit à l'obtention de molécules très ramifiées. Les dextrines font partie des dérivés d'amidon les plus anciens et leur préparation, leurs applications, les différents types de dextrines ainsi que leurs propriétés sont décrits par exemple dans l'ouvrage intitulé "Starch Chemistry and Technology" - Second Edition - Edited by Roy L. WHISTLER - 1984 - Academic Press Inc.

De préférence, on utilise pour la préparation des H.A.H conformes à l'invention des dextrines obtenues par grillage à sec de l'amidon en présence d'un catalyseur acide comme l'acide chlorhydrique. L'acide est ainsi pulvérisé sur l'amidon et le mélange obtenu est préséché, par exemple de 80 à 130°C jusqu'à une teneur en eau inférieure ou égale à environ 5 %. Puis le mélange est "grillé" à une température d'environ 140° à 250°C pendant une durée de 30 minutes à environ 6 heures pour obtenir la dextrine, qui présente en fin de réaction un DE de 0,5 à 10 environ. On peut utiliser, pour la préparation de ces dextrines, n'importe quel type d'amidon, et notamment l'amidon de maïs, l'amidon de pomme de terre, l'amidon de blé ou l'amidon de manioc.

D'après la norme ISO 1227 de 1979, une dextrine est obtenue à partir d'amidon ou de fécule transformés par chauffage à sec avec ou sans addition de petites quantités de réactifs chimiques. Traditionnellement, les dextrines sont classées en deux catégories : les dextrines blanches, dont l'aspect est peu différent de celui de la matière première utilisée, et les dextrines jaunes, produites dans des conditions plus drastiques et dont l'intensité de la coloration peut être corrélée au degré de modification de la structure native. Les quatre types de réaction intervenant lors de la dextrinification sont, aux faibles températures, essentiellement l'hydrolyse des liaisons alpha 1-4, puis aux températures plus élevées des réactions de condensation, de transglycosidation et d'anhydrisation.

Des dextrines telles que celles commercialisées par la Société Demanderesse sous les marques TACKIDEX DF 165, TACKIDEX DF 155, TACKIDEX JO 55 K peuvent être avantageusement utilisées dans le cadre de la présente invention.

La proportion de dextrine et/ou de polyglucose dans le mélange préhydrolysat d'amidon - dextrine soumis à l'hydrolyse enzymatique est généralement dans ce procédé comprise entre 5 et 95 % en poids, ces pourcentages étant exprimés en sec/sec. De préférence, cette proportion est comprise entre 10 et 90 %, et plus préférentiellement encore entre 15 et 75 %.

Dans certains cas, en sélectionnant des types particuliers de dextrines, notamment en sélectionnant des dextrines peu transformées, il est possible d'obtenir une composition d'HAH conforme à l'invention en procédant à l'attaque enzymatique comportant au moins une attaque à la β-amylase directement sur les dextrines pures.

D'autre part un traitement à la seule β-amylase peut être suffisant pour obtenir un H.A.H répondant à la composition définie dans la présente invention. Mais ce traitement à la β-amylase peut être précédé, accompagné ou suivi d'un traitement à l'aide d'une α-amylase. Selon une réalisation particulièrement avantageuse de l'invention, l'hydrolysat obtenu suite à l'action de la β-amylase, et éventuellement de l'α-amylase, est également soumis à l'action d'une enzyme hydrolysant les liaisons 1-6 de l'amylopectine, comme c'est le cas de l'isoamylase et de la pullulanase. Cette action de l'enzyme hydrolysant les liaisons 1-6 de l'amylopectine a l'avantage de ne laisser subsister dans l'hydrolysat obtenu à la suite de son action que les polysaccharides très difficilement digestibles enzymatiquement. L'action de l'enzyme hydrolysant les liaisons 1-6 peut également précéder ou accompagner le traitement par la β-amylase.

Des procédés consistant à hydrolyser enzymatiquement une dextrine ont déjà été proposés dans la littérature.

Ainsi, la demande de brevet européen n° 0 368 451 décrit un procédé consistant essentiellement à dissoudre une pyrodextrine dans l'eau et à faire agir une α-amylase sur la solution résultante. Ce procédé a pour but de retirer l'odeur et le goût indésirables de la pyrodextrine et de conduire à des dextrines contenant des fibres diététiques.

Ainsi, selon le procédé décrit dans cette demande de brevet, une pyrodextrine est dissoute dans l'eau puis hydrolysée avec de l'α-amylase. D'autres enzymes peuvent cependant être ajoutées après le traitement à l'α-amylase : c'est le cas de la transglucosidase, de la β-amylase, de la glucoamylase ou amyloglucosidase. Le produit obtenu à l'issue de ce traitement enzymatique peut alors être hydrogéné. Des monosaccharides et des oligosaccharides peuvent être ajoutés à l'amidon de départ destiné à être dextrinifié, afin d'augmenter la teneur du produit final en dextrine indigestible.

L'objet de la susdite demande de brevet est donc essentiellement de fournir un produit peu digestible, "à basses calories", agissant comme fibre alimentaire diététique, composé essentiellement de polysaccharides peu digestibles. Le contenu des produits exemplifiés en "dextrine indigestible" varie ainsi entre environ 27 % et environ 95 %, et la teneur de ces produits en polysaccharides de degré de polymérisation égal ou supérieur à 4 est comprise entre environ 60 % et environ 92 % en poids.

Un autre procédé d'obtention de libres alimentaires diététiques à partir de dextrines est par ailleurs décrit dans la demande de brevet japonais JP-A-62091501. Ce procédé consiste à traiter à chaud un hydrolysat d'amidon hydrogéné en opérant ce chauffage dans des conditions anhydres à 150-250°C, en présence d'un catalyseur constitué par un acide minéral ou un acide organique.

Tout comme la demande de brevet citée antérieurement, ce document vise donc l'obtention de produits peu digestibles par l'organisme, dits "à basses calories", et agissant dans l'organisme en tant que libres alimentaires. Leur objet est donc très éloigné de l'objet de la présente invention, qui est de préparer un produit édulcorant constitué d'un hydrolysat d'amidon hydrogéné à haut pouvoir sucrant, hypocariogène, aux aptitudes technologiques utilisables aussi bien dans les bonbons, les chewing-gums et les pâtes dentrifice, que dans les boissons et les elixirs pharmaceutiques ou vétérinaires.

D'autre part, on peut souligner que lesdits documents ne décrivent ni ne suggèrent l'utilisation des enzymes hydrolysant les liaisons 1-6 de l'amylopectine. Grâce à l'action de ces enzymes prévue dans le cadre de la présente invention, on obtient des HAH contenant des polysaccharides hydrogénés très peu digestibles et très peu cariogènes, ce qui constitue un avantage important.

L'hydrogénation de l'hydrolysat obtenu à la suite de l'hydrolyse enzymatique du mélange de dextrine et/ou de polyglucose et du pré-hydrolysat d'amidon peut être effectuée de manière connue en soi par hydrogénation sur nickel de Raney ou par hydrogénation sur métaux nobles. Cette hydrogénation est effectuée après purification de l'hydrolysat, par exemple par traitement sur charbon actif, après déminéralisation sur résines cationiques et anioniques, et éventuellement décoloration. L'hydrogénation peut être effectuée par exemple sur catalyseur au nickel de Raney, à une température de 130°C et sous une pression d'hydrogène de 50 bars.

Après hydrogénation, le sirop d'hydrolysat d'amidon hydrogéné obtenu est filtré puis déminéralisé, puis concentré jusqu'à la concentration de commercialisation, qui est généralement comprise entre 70 et 85 Brix environ, soit une matière sèche comprise entre 70 % et 85 %. L'hydrogénation est conduite jusqu'à l'obtention d'un pourcentage de sucres réducteurs résiduels sur matière sèche inférieur à 0,50, de préférence inférieur à 0,25 et plus préférentiellement encore inférieur à 0,20.

La viscosité des hydrolysats d'amidon hydrogénés conformes à l'invention est généralement de 1 000 à 20 000 centipoises à 20°C et à 74 % de matière sèche.

L'une des qualités fondamentales des hydrolysats selon l'invention réside dans leur hypocariogénicité, c'est-à-dire dans leur propriété de provoquer une bien moindre acidification par les bactéries de la bouche que les sucres conventionnels classiques tels que le glucose, le fructose, le saccharose ou les sirops de glucose.

Selon une réalisation tout à fait avantageuse de l'invention, les hydrolysats d'amidon hydrogénés présentent la propriété de pouvoir être qualifiés de non cariogènes selon un test B.

Ce test B avait été mis au point par la Société Demanderesse afin de contrôler le caractère non cariogène des hydrolysats hydrogénés commercialisés à partir de 1978 sous la dénomination LYCASIN 80/55. Ce test simple repose sur la détermination in vitro de l'acidification d'une quantité donnée d'hydrolysat d'amidon hydrogéné après ensemencement du milieu par de la salive. Il repose sur l'appréciation de la chute du pH au cours du temps d'un bouillon de culture contenant le produit à tester, après donc ensemencement avec de la salive provenant de plusieurs donneurs, comparativement à un bouillon de culture témoin ne contenant aucun glucide. Il faut souligner que ce test n'est pas suffisant pour caractériser de façon absolue la non cariogénicité d'un produit, car ses résultats peuvent varier, par exemple, suivant la qualité de la salive utilisée, mais il permet néanmoins d'établir des comparaisons valables entre différents produits.

Le mode opératoire détaillé de ce test est le suivant.

On prépare une série de tubes contenant 10 ml d'un milieu de culture nutritif (milieu trypticase à 2 % de matière sèche) sans sucre à pH 7, et on stérilise ces tubes par passage à l'autoclave à 120°C durant 20 minutes.

Dans une première série de cinq tubes, on introduit 1 ml d'eau stérile pour faire une série témoin.

Dans une deuxième série de cinq tubes, on introduit 1 ml d'une solution à 18 % (P/V) du produit à tester.

Puis on ensemence les cinq tubes de chaque série avec un même volume de 0,2 ml par tube d'une dilution au cinquième de salive humaine obtenue par prélèvement sur cinq donneurs.

On suit alors la formation d'acides par mesure du pH, une première mesure étant effectuée avant incubation et les autres mesures étant effectuées après des incubations à 30°C de respectivement 3, 6, 13, 18 et 21 heures.

Pour qu'un produit puisse être considéré comme non cariogène au sens de ce test B, il faut que la différence de pH observée entre le témoin au bout de 21 heures et le produit à tester au bout de 21 heures, ne soit pas trop prononcée et, dans la pratique, au plus égale à 1 unité de pH.

L'un des grands mérites de la présente invention est de fournir des hydrolysats d'amidon hydrogénés qui présentent la propriété d'être non cariogènes au sens de ce test B, alors qu'ils contiennent pourtant une quantité non négligeable d'oligosaccharides et de polysaccharides.

De préférence, les quantités et les conditions d'action des différentes enzymes citées précédemment et qui peuvent être employées pour la préparation de l'hydrolysat d'amidon destiné à l'hydrogénation subséquente sont choisies parmi les suivantes :

- β-amylase : 100 à 10 000 unités LINTNER par kilo de substrat sec, température de 50°C à 60°C, durée d'action de 30 à 100 heures, pH de 5.0 à 6.0,

- α-amylase : 20 à 2 000 unités KNU (kilo NOVO units) par kilo de substrat sec, pH de 5.0 à 6.0, température de 50°C à 60°C, durée d'action de 16 à 100 heures,
- enzyme hydrolysant les liaisons 1-6 : 150 à 15 000 unités ABM (ABM, Cheshire, England) par kilo de substrat sec, éventuellement en présence de 10 à 500 unités LINTNER de β-amylase par kilo de substrat sec, pH 5.0 à 6.0, température de 50°C à 60°C, durée d'action de 24 à 100 heures.

Les enzymes pouvant être mises en oeuvre peuvent être :

- en ce qui concerne la β-amylase, de la β-amylase microbienne ou d'origine végétale,
- en ce qui concerne l'α-amylase, les α-amylases bactériennes ou fongiques,
- en ce qui concerne celles hydrolysant les liaisons 1-6, celles choisies parmi la pullulanase et l'isoamylase, comme par exemple PULLUZYME d'ABM ou CK 20 L d'AMANO.

Comme matière première pour la préparation du préhydrolysat qui vient en mélange avec la dextrine et/ou le polyglucose, on peut utiliser tous les types d'amidon, dont notamment l'amidon de maïs, l'amidon de pomme de terre, l'amidon de manioc, l'amidon de blé, l'amidon de riz et l'amidon de pois.

Bien entendu, afin de préparer les hydrolysats d'amidon hydrogénés conformes à l'invention, on peut, au lieu d'opérer l'hydrolyse enzymatique sur le mélange de préhydrolysat d'amidon et de dextrine et/ou de polyglucose, effectuer des actions enzymatiques, éventuellement différentes, séparément sur chacun de ces composants, puis mélanger ensuite les produits obtenus dans les proportions voulues afin d'obtenir un hydrolysat d'amidon qui, après hydrogénation, répond à la composition sélectionnée.

De même, il va de soi que la composition en question peut être obtenue par mélange des composants déjà hydrogénés.

L'invention sera mieux comprise à l'aide des exemples qui suivent.

## EXEMPLE 1

Dans des cuves de 25 litres, agitées et thermostatées, on introduit 12 litres d'un sirop composé sur base sèche de :

Exemple 1a :
80 % d'amidon de maïs liquéfié à un D.E de 11,2 par double liquéfaction à l'aide d'alpha amylase thermorésistante TERMAMYL de NOVO et 20 % de dextrine jaune TACKIDEX DF 165 commercialisée par la Société Demanderesse.

Exemple 1b :
55 % de maltodextrine GLUCIDEX 6 B, également commercialisée par la Société Demanderesse, obtenue par hydrolyse enzymatique d'un amidon de maïs jusqu'à un DE égal à 6, et 45 % de la même dextrine jaune TACKIDEX DF 165.

Exemple 1c :
100 % de dextrine jaune TACKIDEX DF 165.

Exemple comparatif 1d :
100 % d'amidon de maïs liquéfié à un DE de 8,0.

Exemple comparatif 1e :
100 % d'amidon de maïs liquéfié à un DE de 6,0.

Tous ces sirops ont été ajustés à une matière sèche voisine de 35 % par simple lecture au Brix-mètre.

Les pH ont été ajustés à 5,5 à l'aide d'acide chlorhydrique ou de soude, les cuves ont été thermostatées à 55°C, puis on y a ajouté :

Exemple 1a :
0,18 °/°° en poids pour poids sec de β-amylase de marque SPEZYME DBA commercialisée par GENENCOR, 2 °/°° en poids pour poids sec de pullulanase de marque PULLUZYME 750 L commercialisée par A.B.M ainsi que 0,2 °/°° d'α-amylase MAXAMYL HT 3000 de GIST.

Après 24 heures de saccharification, on a rajouté 1 °/°° en poids pour poids sec d'α-amylase de marque MAXAMYL.

Au terme de 72 heures, on a stoppé la saccharification par acidification à pH 3,5 et chauffage à 80°C et on a purifié les sirops obtenus, de manière classique, par traitement au charbon actif, déminéralisation sur résines cationiques et anioniques.

Exemple 1b :

0,15 °/°° de β-amylase de marque SPEZYME DBA,

2 °/°° de pullulanase PULLUZYME 750 L.

Après 40 heures de saccharification, on a ajouté 1 °/°° d'α-amylase et on a renouvelé cette addition après 68 heures de saccharification.

On a stoppé la saccharification après 88 heures, puis procédé à la purification du sirop.

Exemple 1c :

0,15 °/°° de β-amylase SPEZYME DBA.

2 °/°° de pullulanase PULLUZYME 750 L.

Après 48 heures de saccharification, on a ajouté 1 °/°° d'α-amylase MAXAMYL HT 3000.

On a stoppé la saccharification au terme de 88 heures puis procédé à la purification du sirop.

Exemple comparatif 1d :

0,2 °/°° en poids/poids de β-amylase SPEZYME DBA, puis après 24 heures de saccharification on y a ajouté 1 °/°° en poids/poids d'α-amylase MAXAMYL HT 3000.

On a stoppé la saccharification au terme de 72 heures puis on a procédé à la purification du sirop.

Exemple comparatif 1e :

0,5 °/°° en poids de β-amylase SPEZYME DBA et 2 °/°° de pullulanase PULLUZYME 750 L.

On a stoppé la saccharification au terme de 48 heures puis on a procédé à la purification du sirop.

Les sirops purifiés obtenus selon les exemples 1b et 1c ont ensuite été traités par addition d'eau oxygénée (8 °/°° en volume/volume d'une solution d'H2O2 à 35 % (v/v) pendant 24 heures à 70°C et pH 9,5). L'eau oxygénée résiduelle a été décomposée par addition d'un peu de catalase puis les sirops ont été désoxygénés sous vide avant d'être à nouveau traités par un peu de charbon actif puis déminéralisés sur lit de résines mélangées.

Tous les sirops ont alors été concentrés à une matière sèche de 40 % environ puis ont été hydrogénés à taux de sucres réducteurs inférieur à 0,5 %.

Le sirop 1c, titrant 15,3 % de maltitol, 22,4 % de polysaccharides de DP > 20, 16,2 % de polysaccharides non hydrolysables selon le test F, 38,2 % de précipité P1 dans l'éthanol et 7,7 % de précipité P2 après digestion enzymatique selon le test A, a alors été additionné de maltitol cristallisé en une proportion telle que ce maltitol rajouté représente 40 % de la matière sèche du sirop, pour constituer l'HAH 1c selon l'invention.

Les HAH obtenus selon l'exemple 1a et l'exemple 1b et l'HAH 1c ont ensuite été filtrés, déminéralisés puis concentrés à une matière sèche d'environ 75 %.

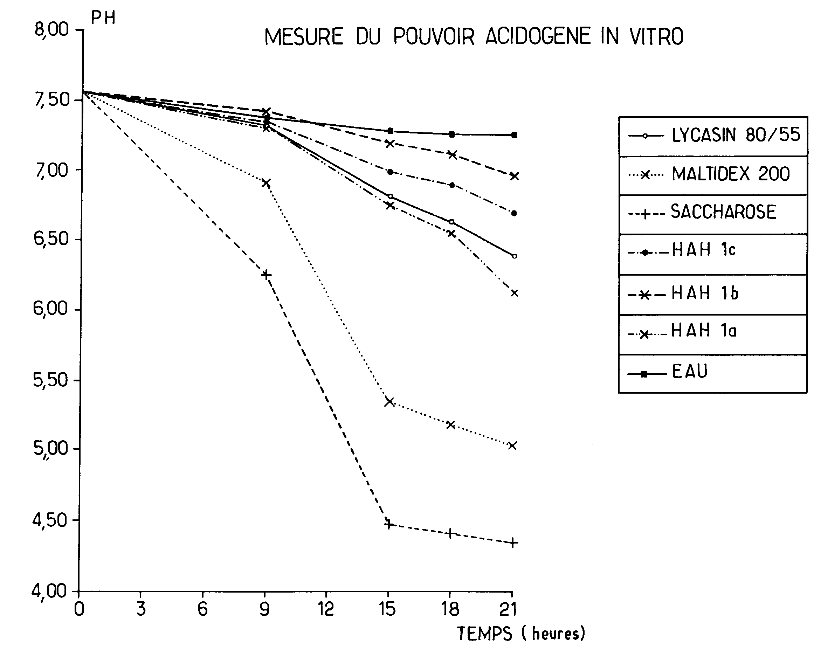

Ces HAH ont révélé les spectres glucidiques suivants à la suite desquels on a fait figurer les résultats obtenus dans le test F et dans le test A (TABLEAU I) et un profil de pH selon le prétest carie B (figure 1)

TABLEAU 1

| | PRODUITS SELON L'INVENTION | | | EXEMPLES COMPARATIFS | |
|---|---|---|---|---|---|
| | HAH 1a | HAH 1b | HAH 1c | HAH 1d | HAH 1e |
| Matière sèche | 75 | 76 | 76 | 75 | 75 |
| Sucres réducteurs (% sur MS) | 0,14 | 0,03 | 0,24 | < 0,1 | < 0,1 |
| DP1 (sorbitol) | 3,4 | 3,1 | 2,5 | 3,7 | 3 |
| DP2 (maltitol et isomaltitol) | 49,6 | 46,3 | 48,9 | 54,5 | 78,4 |
| DP3 | 21,7 | 17,3 | 4,4 | 20 | 10 |
| DP4 | 2,0 | 3,7 | 2,5 | 1,0 | 3 |
| DP5 | 3,1 | 3,0 | 3,0 | 3,8 | 1,7 |
| DP6 | 2,4 | 2,2 | 2,5 | 3,9 | 0,4 |
| DP7 | 1,5 | 2,0 | 2,7 | 2,6 | 0,6 |
| DP8 | 0,9 | 1,9 | 2,6 | 1,7 | 0,3 |
| DP9-20 | 7,8 | 11,5 | 15,5 | 7,5 | 1,7 |
| DP > 20 | 7,3 | 8,7 | 13,8 | 1,0 | 0,9 |
| Polysaccharides non hydrolysables par l'amyloglucosidase (en %) (test F) | 3,1 | 5,0 | 8,7 | 0,1 | 0,0 |
| Précipité P1 dans éthanol (en %) | 10,4 | 12,7 | 23,0 | 0,0 | 0,0 |
| Précipité P2 (%) après digestion enzymatique (test A) | 3,4 | 2,9 | 4,6 | 0,0 | 0,0 |
| Viscosité 20°C (cps) | 4100 | 4800 | 7000 | 2000 | 1800 |

## EXEMPLE 2 : PREPARATION D'HYDROLYSAT D'AMIDON HYDROGENE CONFORME A L'INVENTION AVEC HYDROLYSE ENZYMATIQUE SANS L'ACTION D'UNE ENZYME HYDROLYSANT LES LIAISONS 1-6 DE L'AMYLOPECTINE

Dans une cuve de 25 litres, agitée et thermostatée, on introduit 12 litres d'un sirop composé, sur matière sèche, de 80 % d'amidon de maïs liquéfié à un DE de 12,9 par double liquéfaction à l'aide d'$\alpha$-amylase thermorésistante TERMAMYL 120 L de NOVO et 20 % de TACKIDEX JO 55 K, dextrine obtenue par conversion acide d'amidon de maïs, commercialisée par la Société Demanderesse. Cette dextrine comportait 3,4 % de sucres réducteurs sur matière sèche.

La saccharification a été réalisée en ajoutant audit mélange obtenu 0,2 °/°° en poids/poids d'$\alpha$-amylase MAXAMYL HT 3000 et de 0,18 °/°° poids/poids de $\beta$-amylase SPEZYME DBA. Après 24 heures de saccharification à une température de 55°C et à un pH de 5,5, on ajoute 1 °/°° poids/poids d'$\alpha$-amylase MAXAMYL HT 3000. On stoppe la saccharification après 70 heures et on procède à la purification du sirop. Le sirop ainsi obtenu est ensuite hydrogéné dans les mêmes conditions que celles décrites dans l'exemple 1. L'hydrolysat d'amidon hydrogéné obtenu présente la composition donnée dans le tableau II.

TABLEAU II

| Hydrolysat d'amidon hydrogéné HAH 1f | |
|---|---|
| Matières sèches | 75 % |
| Sucres réducteurs | 0,15 % |
| DP1 (Sorbitol) | 2,2 % |
| DP2 (Maltitol et Isomaltitol) | 51,0 % |
| DP3 | 17,2 % |
| DP4 | 0,9 % |
| DP5 | 2,9 % |
| DP6 | 3,9 % |
| DP7 | 3,2 % |
| DP8 | 2,6 % |
| DP9 | 1,1 % |
| DP10-20 | 10,4 % |
| DP > 20 | 4,7 % |
| Polysaccharides non hydrolysables selon le test F (en %) | 1,2 % |
| Précipité P1 dans l'éthanol (en %) | 12,2 % |
| Précipité P2 (en %) après digestion enzymatique (test A) | 2,9 % |
| Viscosité à 20°C (en cps) | 4300 |

**EXEMPLE 3 PREPARATION D'UN HYDROLYSAT D'AMIDON HYDROGENE DANS LES MÊMES CONDITIONS QUE L'HAH 1a MAIS SANS L'ACTION DE PULLULANASE**

On reprend les mêmes conditions de préparation que celles adoptées pour la préparation de l'hydrolysat d'amidon hydrogéné HAH 1a décrite dans l'exemple 1, hormis le fait qu'au cours de la saccharification on omet d'ajouter les 2 °/°° en poids/poids sec de pullulanase de marque PULLUZYME.

Les autres conditions sont égales par ailleurs. On reprend dans le tableau III ci-après la composition de l'HAH 1g ainsi obtenu.

TABLEAU III

| Matières sèches | 75 % |
|---|---|
| Sucres réducteurs | 0,10 % |
| DP1 (Sorbitol) | 2,3 % |
| DP2 (Maltitol et Isomaltitol) | 49,8 % |
| DP3 | 15,4 % |
| DP4 | 1,0 % |
| DP5 | 3,2 % |
| DP6 | 3,7 % |
| DP7 | 3,1 % |
| DP8 | 2,5 % |
| DP9 | 1,1 % |
| DP10-20 | 11,2 % |
| DP > 20 | 6,8 % |
| Polysaccharides non hydrolysables selon le test F (en %) | 2,8 % |
| Précipité P1 dans l'éthanol (en %) | 13,5 % |
| Précipité P2 (en %) après digestion enzymatique (test A) | 3,1 % |
| Viscosité à 20°C (en cps) | 4900 |

On peut s'apercevoir en comparant les compositions des hydrolysats d'amidon hydrogénés HAH 1a et HAH 1g (ce dernier étant ainsi obtenu sans l'action de la pullulanase) que le pourcentage de DP compris entre 10 et 20 passe de 7,0 à une valeur de 11,2 %, la valeur des DP supérieurs à 20 étant approximativement identique. Le pourcentage de polysaccharides précipitables dans l'éthanol passe de 10,4 à 13,5 %.

**EXEMPLE 4**

Dans une cuve de 25 litres, agitée et thermostatée, on met en suspension dans 20 litres d'eau bouillante environ 2,3 kg de maltose de marque Sunmalt (commercialisé par la Société HAYASHIBARA) et 3,7 kg de polydextrose A (commercialisé par la Société PFIZER). Le pH est ajusté à 5,5 par ajout de soude.

Le mélange est amené à une température de 55°C. On ajoute alors 0,06 % (p/poids sec) d'$\alpha$-amylase fongique MKC LF 40 de Miles, puis on laisse la saccharification se dérouler pendant 48 heures.

Le sirop obtenu est chauffé à 80°C pendant 20 minutes pour inhiber les enzymes, puis purifié par utilisation de charbon actif avant d'être déminéralisé sur résines cationiques fortes et anioniques faibles.

Le sirop est alors concentré à une matière sèche de 40 %, puis hydrogéné comme décrit dans l'exemple 1, avant d'être purifié et concentré.

Le sirop final présente :

-   une teneur en sorbitol de 6,9 %
-   une teneur en maltitol de 36,7 %
-   une teneur en polysaccharides non hydrolysables selon le test F de 2,5 %

La teneur en sucres réducteurs est particulièrement faible puisqu'elle est inférieure à 0,1 %.

**EXEMPLE 5 : EXEMPLE COMPARATIF RELATIF A L'UTILISATION DES SIROPS SELON L'INVENTION DANS LA FABRICATION DE BONBONS AU SUCRE CUIT SANS SUCRE**

Des bonbons du type sucre cuit sans sucre sont préparés à partir des quatre sirops suivants :

EP 0 561 088 B1

- HAH 1a
- HAH 1b
- HAH 1c
- HAH 1d

dont la composition a été donnée dans l'exemple 1.

Ces quatre HAH sont déshydratés par cuisson à pression atmosphérique aux quatre températures suivantes : 140°C, 160°C, 180°C, 200°C, puis coulés dans des moules de téflon après refroidissement vers 130°C. Ils sont ensuite refroidis à température ambiante puis démoulés.

On a comparé les différents bonbons au sucre cuit obtenus, tant du point de vue de leur couleur que de celui de leur stabilité vis-à-vis de l'humidité et de la chaleur.

Avec une cuisson à 140°C, il n'est pas possible d'obtenir avec ces sirops une structure vitrifiée et non collante des bonbons. Les bonbons confectionnés avec l'HAH 1c ont commencé à se colorer à des températures de cuisson atteignant ou dépassant 190°C, mais il n'est pas nécessaire d'atteindre cette température pour fabriquer des bonbons satisfaisants avec cet hydrolysat.

Le tableau IV rassemble les données relatives à la reprise en eau des bonbons obtenus, cette reprise en eau étant effectuée par confinement des bonbons à 66 % d'humidité relative et à 20°C et mesurée par pesée des bonbons. L'hygroscopicité a été exprimée sur une période de 5 jours selon la formule :

$$H5 = \frac{\text{reprise en eau à 13 jours - reprise en eau à 8 jours}}{5}$$

et sur une période de 11 jours selon la formule :

$$H11 = \frac{\text{reprise en eau à 13 jours - reprise en eau à 2 jours}}{11}$$

On constate que les produits obtenus à partir des HAH 1a, 1b et surtout 1c permettent d'obtenir des bonbons au sucre cuit moins hygroscopiques que ceux réalisés avec l'HAH 1d. Les meilleurs résultats sont obtenus avec l'HAH 1c, avec une cuisson à 160°C.

TABLEAU IV

| TEMPERATURE DE CUISSON | PRODUITS SELON L'INVENTION | | | | | | HAH 1d | |
| | HAH 1a | | HAH 1b | | HAH 1c | | | |
| | H5 | H11 | H5 | H11 | H5 | H11 | H5 | H11 |
| 160° | 0,38 | 0,45 | 0,32 | 0,33 | 0,25 | 0,26 | 0,41 | 0,43 |
| 180° | 0,34 | 0,36 | 0,34 | 0,35 | 0,25 | 0,26 | 0,40 | 0,42 |
| 200° | 0,32 | 0,34 | 0,35 | 0,36 | 0,26 | 0,27 | 0,42 | 0,44 |

On a également comparé les différents bonbons au sucre cuit obtenus au niveau de leurs propriétés thermiques traduisant leur propension au fluage (cold flow).

On a donc présenté dans le tableau V les teneurs en eau et les températures de transition vitreuse des différents bonbons obtenus dans le cadre de cet exemple.

La comparaison entre les produits selon l'invention et l'HAH 1d montre que pour des teneurs en maltitol assez proches et voisines de 50 %, les produits selon l'invention permettent de formuler les meilleurs bonbons. On obtient en effet des températures de transition vitreuse élevées avec de faibles températures de cuisson. Par exemple, pour atteindre une température de transition vitreuse de 45°C, il suffit de cuire l'HAH 1c à 160°C alors qu'il faut cuire l'HAH 1d à 200°C, ce qui représente une très nette réduction du coût de production de ces bonbons au sucre cuit.

En outre, les produits obtenus sont moins hygroscopiques puisqu'à température de transition vitreuse égale ils contiennent davantage d'eau.

13

TABLEAU V

| TEMPERATURE DE CUISSON (°C) | PRODUITS SELON L'INVENTION | | | | | | HAH 1d | |
|---|---|---|---|---|---|---|---|---|
| | HAH 1a | | HAH 1b | | HAH 1c | | | |
| | Teneur eau* | Tg* | Teneur eau | Tg | Teneur eau | Tg | Teneur eau | Tg |
| 140° | - | - | 4,20 | 25,3 | 4,20 | 26,6 | - | - |
| 145° | - | - | 3,60 | 29,2 | 3,30 | 35,0 | - | - |
| 150° | - | - | 3,00 | 34,8 | 2,55 | 42,3 | 2,50 | 24,0 |
| 160° | 2,60 | 36,8 | 2,15 | 41,5 | 1,95 | 46,4 | 2,35 | 29,0 |
| 180° | 1,60 | 46,2 | 1,35 | 50,0 | 1,45 | 53,1 | 1,35 | 39,0 |
| 200° | 0,80 | 53,6 | 0,90 | 55,8 | 0,95 | 59,4 | 0,70 | 44,0 |

\* Teneur en eau : teneur en eau finale du bonbon en % (p/p)

\* Tg = température de transition vitreuse en °C

## EXEMPLE 6 : <u>PREPARATION DE SIROPS CONTRE LA TOUX</u>

On a préparé des sirops contre la toux en utilisant les cinq excipients sucrés suivants :

- un sirop de saccharose à 64 % de matière sèche
- l'HAH 1b selon l'invention
- l'HAH 1c selon l'invention
- l'HAH 1d
- le produit MALTIDEX[R] 200, commercialisé par la Société CERESTAR, qui est un hydrolysat d'amidon hydrogéné à 75 % de matière sèche, dont le spectre glucidique est donné dans le tableau VI :

TABLEAU VI

| | |
|---|---|
| SORBITOL | 2,2 % |
| MALTITOL | 53,7 % |
| DP3 | 16,8 % |
| DP4 | 1,1 % |
| DP5 | 0,7 % |
| DP6 | 0,9 % |
| DP7 | 1,2 % |
| DP8 | 2,4 % |
| DP9 | 2,8 % |
| DP 10-20 | 8,3 % |
| DP > 20 | 9,5 % |
| Polysaccharides non hydrolysables selon le test F (en %) | 0,2 % |
| Précipité P1 dans l'éthanol (en %) | 13 % |
| Précipité P2 (en %) après digestion enzymatique selon le test A | 0,7 % |

Les sirops ont été préparés selon les formules suivantes (voir tableau VII) en ajoutant dans un récipient chauffé à 40°C et agité, la glycérine et l'eau, puis la guaifénésine, l'hydrobromure de dextrométorphane, le benzoate de sodium, éventuellement la saccharine, l'excipient sucré, l'acide citrique, le citrate de sodium, le colorant, le menthol dissous dans l'alcool, puis l'arôme. De l'eau a ensuite été ajoutée en quantité suffisante puis les sirops ont été filtrés et embouteillés.

TABLEAU VII

|  | SIROP 1 | SIROP 2 | SIROP 3 | SIROP 4 | SIROP 5 |
|---|---|---|---|---|---|
| DEXTROMETHORPHANE, HB2 | 0,1 | 0,1 | 0,1 | 0,1 | 0,1 |
| GUAIFENESINE | 2,0 | 2,0 | 2,0 | 2,0 | 2,0 |
| ALCOOL | 5,3 | 5,3 | 5,3 | 5,3 | 5,3 |
| ACIDE CITRIQUE ANHYDRE | 0,14 | 0,14 | 0,14 | 0,14 | 0,14 |
| GLYCERINE | 10 | 10 | 10 | 10 | 10 |
| BENZOATE DE SOUDE | 0,1 | 0,1 | 0,1 | 0,1 | 0,1 |
| SACCHARINE |  | 0,2 | 0,2 | 0,2 | 0,2 |
| COLORANT FD ET C ROUGE 40 | 0,003 | 0,003 | 0,003 | 0,003 | 0,003 |
| MENTHOL | 0,01 | 0,01 | 0,01 | 0,01 | 0,01 |
| AROME DE CERISE | 0,3 | 0,3 | 0,3 | 0,3 | 0,3 |
| CITRATE DE SODIUM | 0,38 | 0,38 | 0,38 | 0,38 | 0,38 |
| SIROP DE SACCHAROSE | 87,9 |  |  |  |  |
| HAH 1b |  | 75 |  |  |  |
| HAH 1c |  |  | 75 |  |  |
| HAH 1d |  |  |  | 75 |  |
| MALTIDEX 200 |  |  |  |  | 75 |
| EAU | Q.S.P. 100 ml |  |  |  |  |

Un jury composé de 10 personnes a noté les caractéristiques de ces quatre sirops en termes de goût et de texture.

En termes de goût, tous les sirops ont été jugés de la même manière, la perception de l'intensité sucrée étant comparable et suffisante pour couvrir l'amertume des principes actifs dans tous les cas.

En termes de texture, la rondeur en bouche est beaucoup plus marquée pour les sirops obtenus avec les HAH 1b et 1c selon l'invention. Les sirops obtenus avec les produits HAH 1b, HAH 1c et HAH 1d ne sont pas cariogènes au sens du test B, alors que les sirops confectionnés à l'aide de saccharose et de MALTIDEX[R] 200 le sont.

**EXEMPLE 7 : PREPARATION DE CHEWING-GUMS**

On a préparé des chewing-gums sans sucres cariogènes et sans édulcorants intenses en utilisant la composition HAH 1c selon l'invention.

Pour cela, dans un pétrin comportant deux bras en Z, chauffé préalablement à 45°C et mis en mouvement, on a introduit de façon séquentielle et espacée de 2 minutes :

- 270 g de gomme de base de type 34/42 (commercialisée par la Société DREYFUS) préalablement ramollie à 55°C,
- 200 g de sorbitol poudre de marque NEOSORB [R] P60W commercialisée par la Société demanderesse, et 20 g de glycérine,
- 100 g de mannitol F commercialisé par la Société Demanderesse et 50 g de l'HAH 1c prémélangés,
- 218 g de sorbitol poudre de marque NEOSORB P60 W et 50 g de l'HAH 1c prémélangés et enfin, après 5 minutes de mélange,
- 27,5 g de xylitol broyé de marque XYLISORB [R], commercialisé par la Société demanderesse, 25 g de vitamine C, 2,5 % d'acide citrique, 1,0 g d'une solution aqueuse orangée de colorants et 8,0 g d'arôme mandarine.

Après trois minutes de malaxage, on a laminé et mis en forme la pâte de chewing-gum en utilisant comme poudre de talcage du mannitol F.

On a obtenu de la sorte des chewing-gums de texture souple présentant une aromatisation correcte et ayant, de façon surprenante, un goût sucré plus intense que celui des chewing-gums aux polyols du marché ne contenant pas d'édulcorant intense.

**Revendications**

1. Hydrolysat d'amidon hydrogéné hypocariogène, caractérisé par le fait ou'il présente, les teneurs étant exprimées en poids par rapport à la matière sèche de l'hydrolysat :

   - une teneur de 0,1 à 19 % en sorbitol,
   - une teneur de 35 à 87 % en maltitol,
   - une teneur de 1 à 17 % en polysaccharides non hydrolysables par l'amyloglucosidase dans un test F tel que défini dans la description,

   le complément à 100 % étant constitué par des oligosaccharides et des polysaccharides hydrogénés.

2. Hydrolysat d'amidon hydrogéné hypocariogène selon la revendication 1, caractérisé par le fait qu'il contient de 1,5 à 15 % de polysaccharides non hydrolysables dans le test F.

3. Hydrolysat d'amidon hydrogéné hypocariogène selon la revendication 2, caractérisé par le fait qu'il contient de 2 à 12 % de polysaccharides non hydrolysables dans le test F.

4. Hydrolysat d'amidon hydrogéné hypocariogène, caractérisé par le fait qu'il présente, les teneurs étant exprimées en poids par rapport à la matière sèche de l'hydrolysat :

   - une teneur de 0,1 à 19 % en sorbitol,
   - une teneur de 35 à 87 % en maltitol,
   - une teneur de 1 à 20 % de polysaccharides précipitables dans l'éthanol et non hydrolysables par l'amyloglucosidase dans un test A tel que défini dans la description,

   le complément à 100 % étant constitué par des oligosaccharides et des polysaccharides hydrogénés.

5. Hydrolysat d'amidon hydrogéné hypocariogène selon la revendication 4, caractérisé par le fait qu'il contient de 1 à 10 % de polysaccharides précipitables dans l'éthanol et non hydrolysables dans le test A.

6. Hydrolysat d'amidon hydrogéné hypocariogène selon la revendication 5, caractérisé par le fait qu'il contient de 1,5 à 8 % de polysaccharides précipitables dans l'éthanol et non hydrolysables dans le test A.

7. Hydrolysat d'amidon hydrogéné hypocariogène selon l'une quelconque des revendications 1 à 6, caractérisé par le fait que la teneur en sorbitol est comprise entre 1 et 17 % en poids.

8. Hydrolysat d'amidon hydrogéné hypocariogène selon l'une quelconque des revendications 1 à 7, caractérisé par le fait que la teneur en maltitol est comprise entre 40 et 82 % en poids.

9. Hydrolysat d'amidon hydrogéné hypocariogène selon la revendication 8, caractérisé par le fait que la teneur en maltitol est comprise entre 45 et 77 % en poids.

10. Hydrolysat d'amidon hydrogéné hypocariogène selon l'une quelconque des revendications 1 à 9, caractérisé par le fait qu'il présente une teneur en produits de degré de polymérisation égal à 7 comprise entre 0,2 % et 5 %.

11. Hydrolysat d'amidon hydrogéné hypocariogène selon l'une quelconque des revendications 1 à 10, caractérisé par le fait qu'il présente une teneur en produits de degré de polymérisation supérieur à 20, hydrolysables par l'amyloglucosidase dans le test F, inférieure à 5 %.

12. Procédé de préparation d'un hydrolysat d'amidon hydrogéné hypocariogène selon l'une quelconque des revendications 1 à 11, caractérisé par le fait que l'on mélange un pré-hydrolysat d'amidon d'un dextrose équivalent (DE) compris entre 6 et 20, obtenu par voie acide et/ou enzymatique, à au moins une dextrine et/ou à un polyglucose, et que l'on soumet ce mélange à un traitement enzymatique comportant au moins l'action d'une β-amylase, les conditions de ce traitement étant choisies de façon telle que le DE de l'hydrolysat obtenu soit compris entre 35 et 75 à l'issue dudit traitement, et que l'on hydrogène l'hydrolysat obtenu.

13. Procédé selon la revendication 12, caractérisé par le fait que le traitement enzymatique à la β-amylase est précédé, accompagné ou suivi d'un traitement à l'aide d'une α-amylase.

14. Procédé selon l'une ou l'autre des revendications 12 et 13, caractérisé par le fait que le traitement enzymatique à la β-amylase est précédé, accompagné ou suivi d'un traitement à l'aide d'une enzyme hydrolysant les liaisons 1-6 de l'amylopectine.

15. Procédé selon l'une quelconque des revendications 12 à 14, caractérisé par le fait que les quantités et les conditions d'action des différents enzymes employées pour la préparation de l'hydrolysat d'amidon destiné à l'hydrogénation subséquente sont choisies parmi les suivantes :

- β-amylase : 100 à 10 000 unités LINTNER par kilo de substrat sec, température de 50°C à 60°C, durée d'action de 30 à 100 heures, pH de 5.0 à 6.0,
- α-amylase : 20 à 2 000 unités KNU (Kilo Novo Units) par kilo de substrat sec, pH de 5.0 à 6.0, température de 50°C à 60°C, durée d'action de 16 à 100 heures,
- enzyme hydrolysant les liaisons 1-6 de l'amylopectine : 150 à 15 000 unités ABM (ABM, Cheshire, England) par kilo de substrat sec, éventuellement en présence de 10 à 500 unités LINTNER de β-amylase par kilo de substrat sec, pH 5.0 à 6.0, température de 50°C à 60°C, durée d'action de 24 à 100 heures.

16. Application de l'hydrolysat d'amidon hydrogéné hypocariogène selon l'une quelconque des revendications 1 à 11, comme agent édulcorant ou comme agent de texture dans les produits destinés à être ingérés par les hommes ou les animaux, et en particulier dans les bonbons, les sirops et élixirs alimentaires ou pharmaceutiques, et les chewing-gums.

## Claims

1. A hypocariogenic hydrogenated starch hydrolysate, characterised in that it has, the concentrations being expressed by weight relative to the dry matter of the hydrolysate:

- a concentration of 0.1 to 19% of sorbitol,
- a concentration of 35 to 87% of maltitol,
- a concentration of 1 to 17% of polysaccharides which are not hydrolysed by amyloglucosidase in an F test such as defined in the specification,

the balance for 100% consisting of hydrogenated oligo- and polysaccharides.

2. The hypocariogenic hydrogenated starch hydrolysate as claimed in Claim 1, characterised in that it contains from 1.5 to 15% of polysaccharides which are not hydrolysed in the F test.

3. The hypocariogenic hydrogenated starch as claimed in Claim 2, characterised in that it contains from 2 to 12% of polysaccharides which are not hydrolysed in the F test.

4. A hypocariogenic hydrogenated starch hydrolysate, characterised in that it, has, the concentrations being expressed by weight relative to the dry matter of the hydrolysate:

- a concentration of 0.1 to 19% of sorbitol,
- a concentration of 35 to 87% of maltitol,
- a concentration of 1 to 20% of polysaccharides which can be precipitated in ethanol and are not hydrolysed by amyloglucosidase in an A test such as defined in the specification,

the balance for 100% consisting of hydrogenated oligo- and polysaccharides.

5. The hypocariogenic hydrogenated starch hydrolysate as claimed in Claim 4, characterised in that it comprises from 1 to 10% by weight of polysaccharides which can be precipitated in ethanol and are not hydrolysed in the A test.

6. The hypocariogenic hydrogenated starch hydrolysate as claimed in Claim 5, characterised in that it contains from 1.5 to 8% of polysaccharides which can be precipitated in ethanol and are not hydrolysed in the A test.

7. The hypocariogenic hydrogenated starch hydrolysate as claimed in any one of Claims 1 to 6, characterised in that the sorbitol concentration is between 1 and 17% by weight.

8. The hypocariogenic hydrogenated starch hydrolysate as claimed in any one of Claims 1 to 7, characterised in that the maltitol concentration is between 40 and 82% by weight.

9. The hypocariogenic hydrogenated starch hydrolysate as claimed in Claim 8, characterised in that the maltitol concentration is between 45 and 77% by weight.

10. The hypocariogenic hydrogenated starch hydrolysate as claimed in any one of Claims 1 to 9, characterised in that it has a concentration of products with a degree of polymerisation equal to 7 of between 0.2 and 5%.

11. The hypocariogenic hydrogenated starch hydrolysate as claimed in any one of Claims 1 to 10, characterised in that it has a concentration of products with a degree of polymerization greater than 20, which can be hydrolysed by amyloglucosidase in the F test, of less than 5%.

12. A process for preparing a hypocariogenic hydrogenated starch hydrolysate as claimed in any one of Claims 1 to 11, characterised in that a starch prehydrolysate with an equivalent dextrose DE of between 6 and 20, obtained by the acidic and/or enzymatic route, is mixed with at least one dextrin and/or polyglucose, and that this mixture is subjected to an enzymatic treatment comprising at least the action of a β-amylase, the conditions for this treatment being chosen such that the DE of the obtained hydrolysate is between 35 and 75 at the end of this treatment, and that the hydrolysate obtained is hydrogenated.

13. The process as claimed in Claim 12, characterised in that the enzymatic treatment with β-amylase is preceded, accompanied or followed by a treatment using an α-amylase.

14. The process as claimed in either or Claims 12 and 13, characterised in that the enzymatic treatment with β-amylase is preceded, accompanied or followed by a treatment using an enzyme hydrolysing the 1-6 bonds of amylopectin.

15. The process as claimed in any one of Claims 12 to 14, characterized in that the amounts and conditions for the action of the various enzymes used for preparing the starch hydrolysate intended for subsequent hydrogenation, are chosen from the following :

   - β-amylase: 100 to 10,000 LINTNER units per kilogram of dry substrate, temperature of 50°C to 60°C, duration of action from 30 to 100 hours, pH of 5.0 to 6.0,
   - α-amylase: 20 to 2000 KNU (Kilo Novo Units) units per kilogram of dry substrate, pH of 5.0 to 6.0, temperature of 50°C to 60°C, duration of action from 16 to 100 hours,
   - enzyme hydrolysing the 1-6 bonds of amylopectin: 150 to 15,000 ABM units (ABM, Cheshire, England) per kilogram of dry substrate, optionally in the presence of 10 to 500 LINTNER units of β-amylase per kilogram of dry substrate, pH 5.0 to 6.0, temperature of 50°C to 60°C,

   duration of action from 24 to 100 hours.

16. Application of the hypocariogenic hydrogenated starch hydrolysate as claimed in Claims 1 to 11, as sweetening agent or as texturing agent in products intended to be ingested by humans or animals, and in particular in sweets, food or pharmaceutical syrups and elixirs, and chewing gums.

**Patentansprüche**

1. Hypokariogenes hydriertes Stärkehydrolysat, dadurch **gekennzeichnet,** daß es, ausgedrückt als Gewichtsgehalte, bezogen auf die Trockenmasse des Hydrolysats,

   - einen Gehalt von 0,1 bis 19% Sorbit,
   - einen Gehalt von 35 bis 87% Maltit,

- einen Gehalt von 1 bis 17% durch Amyloglucosidase im F-Test, wie in der Beschreibung definiert, nichthydrolysierbare Polysaccharide enthält, wobei der Rest zu 100% aus hydrierten Oligosacchariden und Polysacchariden besteht.

2. Hypokariogenes hydriertes Stärkehydrolysat nach Anspruch 1, dadurch **gekennzeichnet,** daß es 1,5 bis 15% im F-Test nichthydrolysierbare Polysaccharide enthält.

3. Hypokariogenes hydriertes Stärkehydrolysat nach Anspruch 2, dadurch **gekennzeichnet,** daß es 2 bis 12% im F-Test nichthydrolysierbare Polysaccharide enthält.

4. Hypokariogenes hydriertes Stärkehydrolysat, dadurch **gekennzeichnet,** daß es, ausgedrückt als Gewichtsgehalte, bezogen auf die Trockenmasse des Hydrolysats,

- einen Gehalt von 0,1 bis 19% Sorbit,
- einen Gehalt von 35 bis 87% Maltit,
- einen Gehalt von 1 bis 20% in Ethanol ausfällbare und durch Amyloglucosidase in einem A-Test, wie in der Beschreibung definiert, nichthydrolysierbare Polysaccharide enthält, wobei der Rest auf 100% aus hydrierten Oligosacchariden und Polysacchariden besteht.

5. Hypokariogenes hydriertes Stärkehydrolysat nach Anspruch 4, dadurch **gekennzeichnet,** daß es 1 bis 10% in Ethanol ausfällbare und in dem A-Test nichthydrolysierbare Polysaccharide enthält.

6. Hypokariogenes hydriertes Stärkehydrolysat nach Anspruch 5, dadurch **gekennzeichnet,** daß es 1,5 bis 8% in Ethanol ausfällbare und in dem A-Test nichthydrolysierbare Polysaccharide enthält.

7. Hypokariogenes hydriertes Stärkehydrolysat nach einem der Ansprüche 1 bis 6, dadurch **gekennzeichnet,** daß der Sorbitgehalt zwischen 1 und 17 Gew.-% beträgt.

8. Hypokariogenes hydriertes Stärkehydrolysat nach einem der Ansprüche 1 bis 7, dadurch **gekennzeichnet,** daß der Maltitgehalt zwischen 40 und 82 Gew.-% beträgt.

9. Hypokariogenes hydriertes Stärkehydrolysat nach Anspruch 8, dadurch **gekennzeichnet,** daß der Maltitgehalt zwischen 45 und 77 Gew.-% beträgt.

10. Hypokariogenes hydriertes Stärkehydrolysat nach einem der Ansprüche 1 bis 9, dadurch **gekennzeichnet,** daß es einen Gehalt an Verbindungen mit einem Polymerisationsgrad von 7 zwischen 0,2% und 5% enthält.

11. Hypokariogenes hydriertes Stärkehydrolysat nach einem der Ansprüche 1 bis 10, dadurch **gekennzeichnet,** daß es einen Gehalt an Verbindungen mit einem Polymerisationsgrad über 20, die in dem F-Test durch Amyloglucosidase hydrolysierbar sind, von kleiner als 5% besitzt.

12. Verfahren zur Herstellung eines hypokariogenen hydrierten Stärkehydrolysats nach einem der Ansprüche 1 bis 11, dadurch **gekennzeichnet,** daß man ein Vorhydrolysat eines Dextrosestärkeäquivalents (DE) zwischen 6 und 20, erhalten durch Säure und/oder enzymatisch, mit mindestens einem Dextrin und/oder einer Polyglucose vermischt, und daß man dieses Gemisch einer enzymatischen Behandlung, umfassend mindestens die Wirkung einer β-Amylase, unterwirft, wobei die Bedingungen dieser Behandlung so ausgewählt sind, daß das DE des so erhaltenen Hydrolysats zwischen 35 und 75 am Ende der Behandlung beträgt, und daß man das so erhaltene Hydrolysat hydriert.

13. Verfahren nach Anspruch 12, dadurch **gekennzeichnet,** daß man vor, begleitend oder nach der enzymatischen Behandlung mit β-Amylase mit einer α-Amylase behandelt.

14. Verfahren nach einem der Ansprüche 12 oder 13, dadurch **gekennzeichnet,** daß man vor, begleitend oder nach der enzymatischen Behandlung mit β-Amylase mit einem Enzym, das die 1-6-Bindungen des Amylopectins hydrolysiert, behandelt.

15. Verfahren nach einem der Ansprüche 12 bis 14, dadurch **gekennzeichnet,** daß man die Mengen und die Wirkbedingungen der verschiedenen Enzyme, die zur Herstellung des Stärkehydrolysats, das anschließend hydriert werden soll, verwendet werden, aus den folgenden auswählt:

- β-Amylase: 100 bis 10.000 LINTNER-Einheiten pro Kilo Trockensubstrat, Temperatur 50°C bis 60°C, Wirkdauer 30 bis 100 Stunden, pH-Wert 5,0 bis 6,0.,
- α-Amylase: 20 bis 2.000 KNU-Einheiten (Kilo Novo Units) pro Kilo Trockensubstrat, pH-Wert 5,0 bis 6,0, Temperatur 50°C bis 60°C, Wirkdauer 16 bis 100 Stunden,
- Enzym, das die 1-6-Bindungen des Amylopectins hydrolysiert: 150 bis 15.000 ABM-Einheiten (ABM, Cheshire, England) pro Kilo Trockensubstrat, gegebenenfalls in Gegenwart von 10 bis 500 LINTNER-Einheiten β-Amylase pro Kilo Trockensubstrat, pH-Wert 5,0 bis 6,0, Temperatur 50°C bis 60°C, Wirkdauer 24 bis 100 Stunden.

16. Verwendung des hypokariogenen hydrierten Stärkehydrolysats nach einem der Ansprüche 1 bis 11 als Süßstoff oder als Texturstoff in Produkten, die für den menschlichen oder tierischen Verzehr bestimmt sind, und insbesondere in Bonbons, Sirupen und Lebensmittel- oder Arzneimittelelixieren und Kaugummis.

MESURE DU POUVOIR ACIDOGENE IN VITRO

Légende:
- LYCASIN 80/55
- MALTIDEX 200
- SACCHAROSE
- HAH 1c
- HAH 1b
- HAH 1a
- EAU

PH (axe vertical): 4,00 — 8,00

TEMPS ( heures ) (axe horizontal): 0, 3, 6, 9, 12, 15, 18, 21

EP 0 561 088 B1